# EUROPEAN PATENT SPECIFICATION

(11) **EP 1 882 741 B1**
(45) Date of publication and mention of the grant of the patent: **30.06.2010**
(21) Application number: 06291205.0
(22) Date of filing: 25.07.2006
(51) Int. Cl.: C12N 15/63, C07K 14/35, A61K 35/74, A61K 39/04

(54) **Recombinant Mycobacterium strain expressing a mycobacterial FAP protein under the control of a promoter active under hypoxia and its application for cancer therapy**
Rekombinanter Mycobakteriumstamm, der ein FAP Protein aus Mycobakterium unter der Kontrolle eines Promotors, der unter hypoxischen Konditionen aktiv ist, exprimiert, und dessen Verwendung zur Tumortherapie
Souche de Mycobacterium exprimant une proteine FAP sous le contrôle d'un promoteur qui est actif en conditions d' hypoxie et ses applications pour la thérapie du cancer

(43) Date of publication of application: 30.01.2008
(73) Proprietor: INSTITUT PASTEUR, 75015 Paris (FR)
(72) Inventor: Marchal, Gilles, 94200 Ivry-sur-Seine (FR); Abolhassani, Mohammad, 75015 Paris (FR)
(74) Representative: Goulard, Sophie

(56) References cited:
- WO-A-96/20276
- LAQUEYRERIE A ET AL: "CLONING, SEQUENCING AND EXPRESSION OF THE APA GENE CODING FOR THE MYCOBACTERIUM TUBERCULOSIS 45/47-KILODALTON SECRETED ANTIGEN COMPLEX" INFECTION AND IMMUNITY, AMERICAN SOCIETY FOR MICROBIOLOGY. WASHINGTON, US, vol. 63, no. 10, 1 October 1995 (1995-10-01), pages 4003-4010, XP002001489 ISSN: 0019-9567
- YUAN ET AL: "Stationary phase-associated protein expression in Mycobacterium tuberculosis: function of the mycobacterial alpha-crystallin homolog" JOURNAL OF BACTERIOLOGY, WASHINGTON, DC, US, vol. 178, no. 15, August 1996 (1996-08), pages 4484-4492, XP002086106 ISSN: 0021-9193
- FLORCZYK MATTHEW A ET AL: "A family of acr-coregulated Mycobacterium tuberculosis genes shares a common DNA motif and requires Rv3133c (dosR or devR) for expression." INFECTION AND IMMUNITY, vol. 71, no. 9, September 2003 (2003-09), pages 5332-5343, XP002401486 ISSN: 0019-9567
- ZHAO WEICHENG ET AL: "Role of a bacillus Calmette-Guerin fibronectin attachment protein in BCG-induced antitumor activity" INTERNATIONAL JOURNAL OF CANCER, vol. 86, no. 1, 1 April 2000 (2000-04-01), pages 83-88, XP002401487 ISSN: 0020-7136
- LEE CHI-FENG ET AL: "Treatment of bladder carcinomas using recombinant BCG DNA vaccines and electroporative gene immunotherapy." CANCER GENE THERAPY, vol. 11, no. 3, March 2004 (2004-03), pages 194-207, XP002401488 ISSN: 0929-1903
- LOSA ET AL: "LOW DOSE BACILLUS CALMETTE-GUERIN FOR CARCINOMA IN SITU OF THE BLADDER: LONG-TERM RESULTS" JOURNAL OF UROLOGY, BALTIMORE, MD, US, vol. 163, no. 1, January 2000 (2000-01), pages 68-72, XP005556180 ISSN: 0022-5347
- GRECO O. ET AL: 'Novel chimeric gene promoters responsive to hypoxia and ionizing radiation.' GENE THERAPY vol. 9, 2002, pages 1403 - 1411

### SEQUENCE LISTING

<110> INSTITUT PASTEUR
   MARCHAL, Gilles
   ABOLHASSANI, Mohammad
<120> RECOMBINANT MYCOBACTERIUM STRAIN EXPRESSING A MYCOBACTERIAL FAP PROTEIN UNDER THE CONTROL OF A PROMOTER ACTIVE UNDER HYPOXIA AND ITS APPLICATION FOR CANCER THERAPY
<130> HLPcp226-131
<160> 6
<170> PatentIn version 3.3
<210> 1
   <211> 33
   <212> DNA
   <213> Artificial
<220>
   <223> Forward primer
<400> 1
   catgccatgg tacaggtgga ccccaacttg aca 33
<210> 2
   <211> 33
   <212> DNA
   <213> Artificial
<220>
   <223> Reverse primer
<400> 2
   ttaggatccg gccggtaagg tccgctgcgg tgt 33
<210> 3
   <211> 29
   <212> DNA
   <213> Artificial
<220>
   <223> Primer Bamhspx F
<400> 3
   ttaggatccg tccggcatga tcaacctcc 29
<210> 4
   <211> 30
   <212> DNA
   <213> Artificial
<220>
   <223> Primer NcohspX R
<400> 4
   catgccatgg ggtggccatt tgatgcctcc 30
<210> 5
   <211> 29
   <212> DNA
   <213> Artificial
<220>
   <223> Primer BamRv2623 F
<400> 5
   ttaggatccg ggccatggac tggtcgtcg 29
<210> 6
   <211> 30
   <212> DNA
   <213> Artificial
<220>
   <223> Primer NcoRV2623 R
<400> 6
   catgccatgg acatcgcggt cctcctgtcg 30

## Description

The invention relates to a recombinant Mycobacterium strain expressing a polynucleotide fragment encoding a mycobacterial FAP protein under the transcriptional control of a promoter active under hypoxia conditions and its use for the prevention and the treatment of epithelial tumors.

Bladder cancer is among the top five cancers in men and is three to four time less frequent in women. Over 80 % of these cancers are superficial transitional cell carcinomas. Intravesical immunotherapy with bacillus Calmette-Guerin (BCG) is, today, the reference for treatment and prophylaxis of superficial transitional cell carcinoma of the urinary bladder; it is the most effective therapy for preventing superficial tumor recurrences and treating residual tumors of the bladder after transurethral tumor resection (Lamm et al., N. Engl. J. Med., 1991, 325, 1205-).

While the anti-tumor effect of BCG is well-established, the mechanism through which it occurs is less clear. Some studies suggest that the cell mediated immune response to BCG plays a critical role in the BCG anti-tumor effect. At the same time, other studies identify a direct anti-tumor effect of BCG. The relative contribution of these two possible mechanisms to BCG's clinical anti-tumor activity remains unknown.

The instillation of BCG into the bladder provokes a complex inflammatory response during which lymphocytes and various cytokines are released into the urine. Local granulomas formed in the suburothelial stroma are proposed to play a key role in the control of carcinoma cells (Alexandroff et at, The Lancet, 1999, 353, 1689-1694). NK cells have been hypothesized to mediate a part of this control. The production of different cytokines (IL-2, IL-12, IL-18 and interferons α) is over-expressed after BCG therapy and may activate NK cells (Suttmann et al., The Journal of Urology, 2004, 172, 1490-1495). However, the intense immune responses observed in some patients did not correlate with a better disease control and the immunological mechanisms allowing the proliferation control of carcinoma cells are still unclear.

A number of studies demonstrate that BCG exerts a direct anti-proliferative effect on human urothelial carcinoma cells. The alanine and proline rich secreted protein APA (also named FAP-B, antigen MPT-32, 45-kDa glycoprotein, or 45/47-kDa antigen) of the mycobacterial fibronectin attachment protein (FAP) family is a necessary protein for *in vivo* BCG attachment to the bladder epithelial cells and mediation of BCG-induced anti-tumor activity (Zhao et al., Int. J. Cancer, 2000, 86, 83-88). Mycobacterial FAP proteins constitute a family of highly homologous proteins that bind fibronectin in a unique manner. FAP proteins from at least five mycobacterial species including *M. leprae* (FAP-L), *M. avium* (FAP-A), *M. bovis* BCG (FAP-B; GenBank accession number AF013569), *M. smegmatis* (FAP-S) and *M. tuberculosis* (APA, antigen MPT-32, 45-kDa glycoprotein, or 45/47-kDa antigen; EMBL accession number X80268) have been cloned and characterized. The fibronectin binding region (positions 269-292 of FAP-A) contains a conserved RWFV tetrapeptide (positions 273 to 276 of FAP-A) that is necessary for fibronectin binding function. The minimal binding sequence is the 12 amino acid peptide, 269-280 (positions 269 to 280 of FAP-A; Zhao et al., The Journal of Biological Chemistry, 1999, 274, 4521-4526). The APA protein functions as an opsonin, linking BCG to cell surface integrins, via a fibronectin bridge. FAP-mediated BCG adherence to the urothelial carcinoma surface α5β1 integrin, which is the predominant FAP receptor on urothelial cells, induces signaling and gene transactivation pathways involving NF-κB and AP-1. Cell-cycle arrest at the G1/S interface, rather than apoptosis, is the mechanism contributing to BCG's anti-proliferative effect (Chen et al., BMC Urology, 2005, 5:8).

However, the use of BCG for bladder cancer does not come without drawbacks, both in terms of efficacy and toxicity. First, the response to BCG is unpredictable and not linear. As a result, thirty percent of patients are BCG refractory and there are currently no reliable prognostic factors that accurately predict treatment success or failure. In addition, the long-term durability of response to BCG is limited and the use of maintenance (three weekly treatments every three to six months) or booster therapy (once a month) for up to 36 months is necessary to reduce recurrence of bladder cancer.

Second, BCG has side effects. Most patients experience local symptoms of cystitis including frequency, urgency, dysuria and occasional haematuria. Mild systemic symptoms of high temperature, malaise, and a transient influenza-like illness are also common. Severe side-effects occur in 5 % of patients, roughly 10 % of which involve frank BCG sepsis (Alexandroff *et al*., precited).

Recent advances in recombinant mycobacteria technology, have open new horizons for bladder cancer immunotherapy. Several approaches have been suggested to produce a recombinant alternative to BCG which is less toxic and/or more effective than the existing BCG (Alexandroff *et al*., precited):
- a recombinant BCG expressing human cytokines of interest such as interleukin 2, interferon gamma, tumor necrosis factor alpha; it is expected that this recombinant BCG should be more immunopotent and thus could lead to the use of lower doses of BCG and minimize the chance of systemic BCG infection,
- a recombinant strain of BCG which is less virulent or which incorporates a suicide gene or antimicrobial drug susceptibilies that are less toxic and more effectice than conventional antituberculosis drugs; it is expected that these recombinant BCG should produce less side effects.

In addition, it has been suggested to use a non-pathogenic strain of mycobacteria, such as *M. smegmatis*.

However, to date, none of these approaches have provided an alternative to BCG immunotherapy. It is in this context that the invention was made.

Knowing that the P_{O2} in urine is low (Leonhardt et al., New Engl. J. Med., 1963, 269, 115-121), i.e. the local P_{O2} of the surface of the bladder epithelium where carcinoma cells are growing, the inventors have engineered a recombinant BCG (BCG Apa⁺⁺) overexpressing the APA protein under hypoxic conditions. In this construct, expression of the APA open reading frame is driven by the mycobacterial α-crystallin promoter. The α-crystallin is a protein which is specifically synthesized during *M. tuberculosis* late exponential and stationary phase growth *in vitro,* following a shift to oxygen-limiting conditions, and may play a role in long-term survival of *M*. *tuberculosis, in vivo* (Yuan et al., J. Bacteriol., 1996, 178, 4484-4492). In *vivo* administration of BCG Apa⁺⁺, leads to overexpression of the APA protein in tissues were the partial pressure of oxygen is low, such as the bladder (Leonhardt et al., N. England J. Medecine, 1963, 269, 115-121). As a result, BCG Apa⁺⁺ is more active than wild-type BCG to control human bladder carcinoma cells proliferation, both *in vitro* and *in vivo*. This recombinant BCG should lower the doses of BCG used and minimize the side-effects associated with BCG immunotherapy.

Therefore, the invention relates to a recombinant vector comprising a polynucleotide fragment encoding a mycobacterial FAP protein under the transcriptional control of a promoter sequence which is active under hypoxia conditions.

### Definitions

- by "vector" is intended a nucleic acid molecule capable of transporting another nucleic acid to which it has been linked, into a host cell.
- by "expression vector" is intended a vector capable of directing the expression of a cloned polynucleotide.
- by "promoter sequence" is intended a nucleic acid sequence that is necessary to permit an RNA polymerase to initiate transcription of a polynucleotide. Said promoter may also include regulatory sequences (enhancer, repressor), that serve to enhance or repress transcription.
- by "transcriptional control" is intended that the polynucleotide encoding the mycobacterial FAP protein is operatively linked to the promoter (*i*.*e.* at a position allowing transcription of said polynucleotide and translation of the corresponding protein to occur in the host cells modified by the recombinant expression vector.
- by "hypoxia" is intended a partial pressure of oxygen which is inferior to 30 mm Hg, as determined by conventional techniques, for example by using a polarographic electrode.
- by "mycobacterial FAP protein" is intended a protein encoded by the *apa* gene of *M. tuberculosis* (EMBL accession number X80268), or its homolog from other mycobacteria species, including for example the FAP-B gene of *M. bovis* (GenBank AF013569). The APA protein amino acid sequence corresponds to the SwissProt accession number Q50906.
- by " *acr* gene" or "hspX gene" is intended the gene encoding the alpha-crystallin heat shock protein homolog HSPX (14 kDa antigen; HSP16.3). By reference to *M. tuberculosis* H37Rv sequence (accession number NC_000962.2), the hspX gene (Gene ID: 887579; locus tag Rv2031c) corresponds to positions 2278498 to 2278932 on the minus strand. The hspX gene corresponds also to GenBank accession number 579751. By reference to M. bovis sequence (accession number AF2122/97), the hspX gene (Gene ID: 1094114; locus tag MB2057C) corresponds to positions 2262845 and 2262411 (5' to 3'); said gene is on the DNA minus strand and the amplified promoter region is included between positions 2263059 and 2262846(5' to 3')

According to the present invention the FAP coding sequence may be the FAP gene, the FAP protein coding sequence (CDS), corresponding to the full-length FAP open reading frame (ORF), or a truncated coding sequence, wherein the N-terminal signal sequence has been deleted.

In a first embodiment, the invention features a recombinant vector, wherein said polynucleotide fragment encodes a *Mycobacterium tuberculosis* FAP protein (*M. tuberculosis* APA protein).

In a second embodiment, the invention features a recombinant vector, wherein the promoter is a mycobacterial promoter which is active in hypoxic conditions. Examples of such promoters are described in Florczyk et al., Infect. Immun., 2001, 69, 5777-5785 and Florczyk et al., Infect. Immun., 2003, 71, 5332-5343. Among said promoters, the one specified here above may advantageously be used (see chapter definitions, hspX gene); another promoter may also be advantageously used: it corresponds to the promoter of the Rv2623 gene (accession number of the corresponding protein of *M. tuberculosis:* gi2104288); the primers used to amplify said promoter are those described in Table 1 in Florczyk et al., (Infect. Immun., 2003, precited), modified in view to add the restriction sites BamHI or NcoI at the 5'end

Preferably, the mycobacterial promoter is the promoter of the *acr* gene (HSPX promoter); expression of the alpha-crystallin heat shock protein homolog HSPX (14 kDa antigen; HSP16.3) encoded by the mycobacterial *acr* gene, is specifically induced to very high level under hypoxic conditions.

More preferably, the vector comprises a polynucleotide fragment encoding the *M. tuberculosis* APA protein under the transcriptional control of the HSPX promoter.

A vector according to the present invention comprises, but is not limited to, a YAC (yeast artificial chromosome), a BAC (bacterial artificial), a phage, a phagemid, a cosmid, a viral vector, a plasmid, a RNA vector or a linear or circular DNA or RNA molecule which may consist of chromosomal, non chromosomal, semisynthetic or synthetic nucleic acids. Preferred vectors are those capable of autonomous replication and/or expression of nucleic acids to which they are linked. One type of preferred vector is an episome, i.e., a nucleic acid capable of extra-chromosomal replication. In general, expression vectors of utility in recombinant DNA techniques are often in the form of "plasmids" which refer generally to circular double-stranded DNA loops which, in their vector form are not bound to the chromosome. Large numbers of suitable vectors are known to those of skill in the art.

The vector according to the present invention comprises an expression cassette, wherein the sequence encoding the FAP protein is placed under control of appropriate transcriptional and translational control elements to permit production or synthesis of said protein. More particularly, the vector may comprise additional features which are well-known in the art, including:
- a cloning site located downstream of the promoter region, for insertion of the ORF. The ATG initiation codon and/or the stop codon of the ORF may be included in a restriction site for allowing insertion of the protein coding sequence in frame with the vector. Optionally, the vector may comprise a multiple cloning site, comprising multiple adjacent cloning sites allowing any insert to be cloned in the vector in frame with any reading frame in the vector (fusion proteins). For example, the 5' or 3' end of the ORF can be fused to a sequence encoding a tag. The tag may be used for the purification (polyhistidine sequence) or the detection (B epitope, fluorescent protein) of the expressed protein. Optionally, the 5' or 3' end of the ORF and the sequence encoding the tag are separated with a cleavage site for a protease/endopeptidase sequence to allow removal of the tag.
- at least one selectable marker, for example an auxotrophy marker, an antibiotic resistance gene (tetracycline, rifampicin, ampicillin, kanamycin resistance) or a mercury resistance gene, for the selection of recombinant bacteria.
- a transcription termination signal (terminator), donwnstream of the ORF; to cause RNA polymerase from the host to terminate transcription of the transcript generated by the promoter.
- at least one origin of replication for the maintenance of the vector in the host.

In a fourth embodiment, the invention features a vector which is a plasmid (double-stranded circular DNA). Preferably, said plasmid contains a bacterial origin of replication and an antibiotic resistance gene. In particular, the invention features a plasmid, identified as pYAPA2031 , wherein a polynucleotide fragment encoding the *M. tuberculosis* APA protein under the transcriptional control of the HSPX promoter is inserted in the *EcoR*V site of the pYUB295 plasmid (Dussurget et al., Infect. Immun., 2001, 69, 529-533; Gomez et al., Mol. Microbiol., 1998, 29, 617-628).

The invention concerns also an expression cassette derived from the recombinant vector according to the present invention, consisting of a polynucleotide fragment comprising the promoter sequence which is active under hypoxia conditions operatively linked to the polynucleotide fragment encoding a mycobacterial FAP protein, as defined above. Said expression cassette may further comprise additional features as defined above (cloning site, tag, downstream termination signal).

The invention relates also to a host cell which is genetically modified by a recombinant vector as defined above (recombinant cell).

Particularly, the invention relates to bacteria, preferably mycobacteria transformed by a recombinant vector as defined above.

Preferred mycobacteria include those of *M. tuberculosis* complex, such as *M. bovis* BCG strains, for example the Pasteur BCG strain. Other preferred mycobacteria are those which are fast growing and non pathogenic for humans such are for example, *Mycobacterium smegmatis, Mycobacterium fortuitum* and *Mycobacterium microti.*

In particular, the invention features a recombinant *Mycobacterium bovis* BCG strain, stably transformed with the plasmid pYAPA2031, deposited at the Collection Nationale de Cultures de Microorganismes, 25 rue du Docteur Roux, 75724 Paris Cedex 15, on July 21 2006, under accession number 1-3659, identified as BCG Apa⁺⁺.

The invention relates also to an antitumoral composition comprising a suitable amount of a recombinant vector or host cell as defined above, in an acceptable carrier, such as a stabilizer, a buffer and the like.

The composition according to the present invention may comprise living or killed vector/cell. The killed vector/cell compositions are prepared by any means, known to those of ordinary skill in the art.

A pharmaceutical composition or formulation refers to a form suitable for administration (oral, topical, by injection or inhalation) into a subject, for example a mammal, and in particular a human. Suitable forms, in part, depend upon the use or the route of entry. A preferred formulation is for topical administration, more preferably for intravesical, intravaginal, rectal administration, or for inhalation.

A pharmaceutically effective dose is that dose required to prevent, inhibit the occurrence or treat (alleviate a symptom to some extent, preferably all the symptoms) of a disease or state. The pharmaceutically effective dose of the vector/host cell depends upon the composition used, the route of administration, the type of mammal being treated, the physical characteristics of the specific mammal under consideration, concurrent medication, and other factors, that those skilled in the medical arts will recognize. Generally, the effective dose of recombinant mycobacteria for human, is between 10⁶ and 10¹⁰CFU (Colony Forming Unit) or CFU equivalent.

Preferably, the tumor is an epithelial tumor. More preferably, the tumor is a superficial tumor. In particular, the tumor is a transitional cell carcinoma of the bladder or a lung, colon or cervix carcinoma.

In a first embodiment, the antitumoral composition according to the present invention, comprises a recombinant mycobacteria strain as defined above, in particular the BCG Apa⁺⁺ strain.

Preferably, the recombinant mycobacteria strain is killed, more preferably by extended freeze-drying. The method of preparation of extended-freeze-drying mycobacteria strains is described in the PCT International Application WO 03049752.

In a second embodiment, the antitumoral composition according to the present invention, further comprises an immunostimulatory agent.

The invention relates also to the use of a recombinant vector or host cell as defined above for the manufacture of a medicament intended for the prevention or the treatment of a tumor in a subject.

The invention relates also to a product containing a recombinant vector or a host cell as defined above, and an immunostimulatory agent, as combined preparation for simultaneous, separate or sequential use in antitumoral therapy.

The invention relates also to a method for preventing or treating a tumor in a subject, comprising: administering to the subject, an effective amount of a composition, as defined above, by any appropriate means. Particularly, the composition is administered intravesically, intravaginally, rectally, or by inhalation and comprises between 10⁶ and 10¹⁰ CFU (Colony Forming Unit) or CFU equivalent of recombinant mycobacteria.

The invention relates also to the use of the expression cassette or recombinant vector, as defined above for the preparation of a modified host cell, preferably a recombinant mycobacteria strain wherein the expression cassette is integrated in the genome of the recombinant mycobacteria.

The practice of the present invention will employ, unless otherwise indicated, conventional methods of microbiology, molecular biology and immunobiology within the skill of the art. Such techniques are explained fully in the literature.

The recombinant vector according to the invention is constructed and introduced in a host cell by the well-known recombinant DNA and genetic engineering techniques using classical methods, according to standard procedures as those described in: Current Protocols in Molecular Biology (Frederick M. A USUBEL, 2000, Wiley and son Inc, Library of Congress, USA*) and* Molecular Cloning: A Laboratory Manual, Third Edition, (Sambrook et al, 2001, Cold Spring Harbor, New York: Cold Spring Harbor Laboratory Press).

In addition to the preceding features, the invention further comprises other features which will emerge from the description which follows, which refers to examples illustrating the BCG recombinant strain and its use according to the invention, as well as to the appended drawings in which:

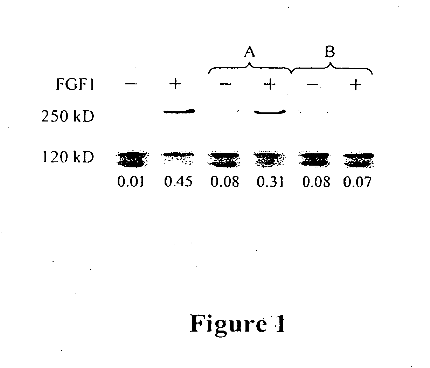

- Figure 1 illustrates the inhibition of FGFR-3 activation by BCG strains, analyzed by immunoblotting. T24 cells were incubated 48 h without bacteria or with 10⁶ CFU/ml BCG vaccine (A) or BCG Apa⁺⁺ (B), cooled at 4 °C during 15 min, and treated for 2 h at 4 °C in the absence (-) or presence (+) of 50 ng/ml of FGF-1. After covalent cross-linking with bis-(sulfosuccinimidyl)-suberate and disuccinimidyl-suberate, the cells were lysed, immunoprecipitated with anti-FGFR-3 C-terminal antibody and probed on an immunoblot with a polyclonal antibody to the cytoplasmic domain of FGFR-3. The values represent the density obtained for the dimers (∼ 250 kDa) relative to the sum of the density of monomers (∼ 120 kDa) and dimers.

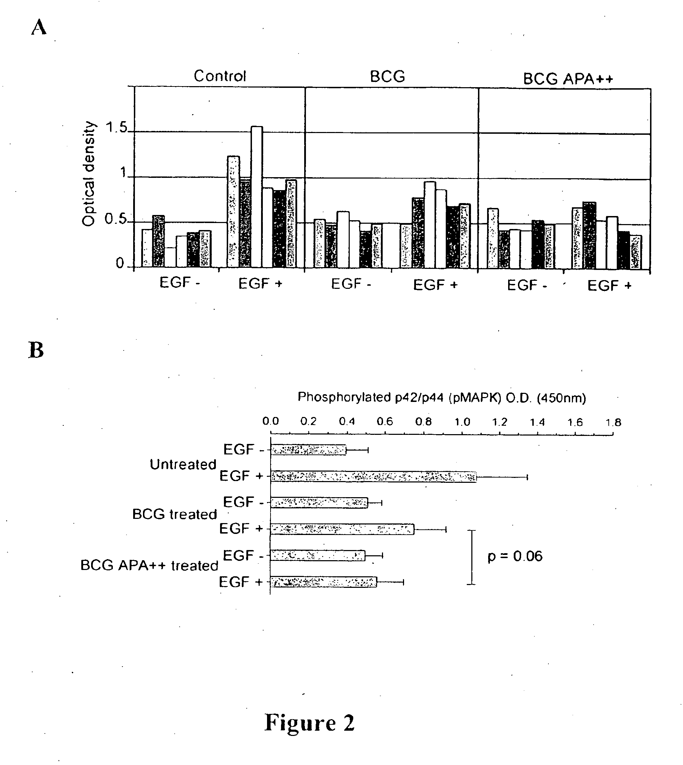

- Figure 2 illustrates the stimulation of MAPK pathway by BCG strains. T24 cells were incubated 48 h without bacteria or with 10⁶ CFU/ml BCG vaccine or BCG Apa⁺⁺, incubated for 15 minutes in the absence (-) or presence (+) of 100 ng/ml of EGF and lysed. The presence of phosphorylated mitogen-activated protein kinase p42/p44 (pMAPK) was determined by ELISA.**A**. Data reported well by well. **B.** Data reported as mean ± s.e.m.

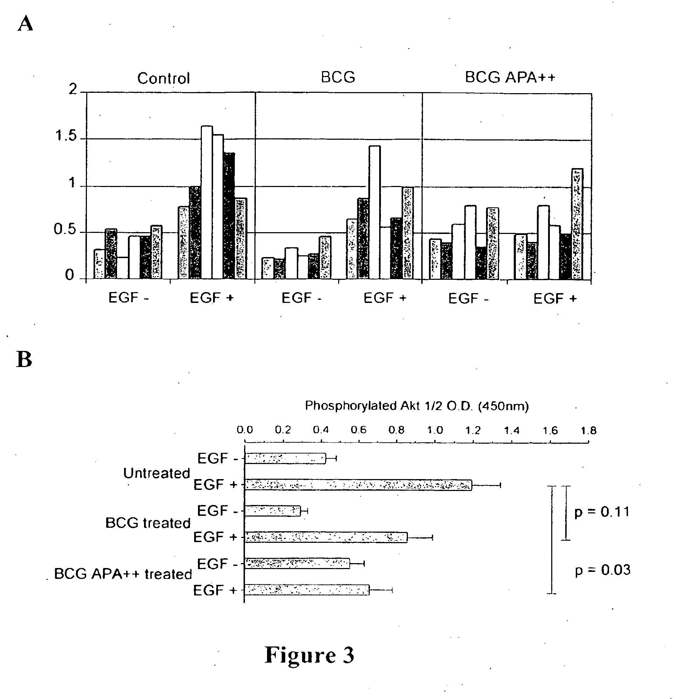

- Figure 3 illustrates the stimulation of Aktl-2 pathway by BCG strains. T24 cells were incubated 48 h without bacteria or with 10⁶ CFU/ml BCG vaccine or BCG Apa⁺⁺, incubated for 15 minutes in the absence (-) or presence (+) of 100 ng/ml of EGF and lysed. The presence of phosphorylated Akt 1-2 was determined by ELISA. **A.** Data reported well by well. **B**. Data reported as mean ± s.e.m.

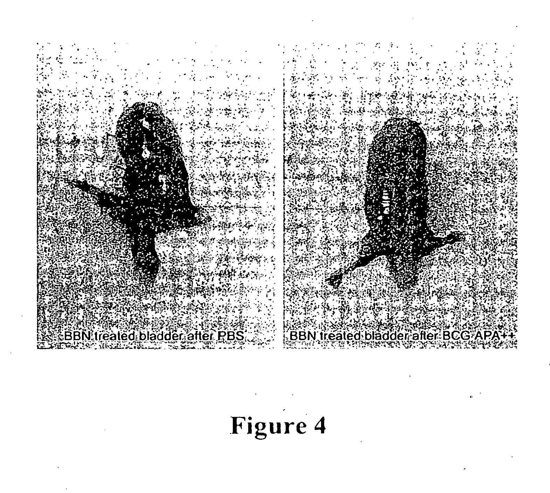

- Figure 4 is a view of bladders collected from rat receiving BBN and treated with PBS (left panel) or BCG Apa⁺⁺ (right panel).

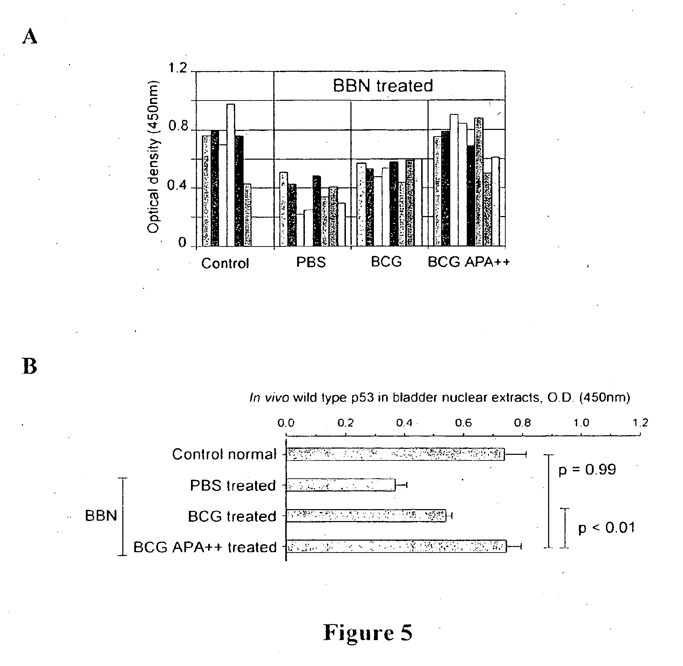

- Figure 5 illustrates the activation of wild type p53 in nucleus of cells of BBN-induced bladder tumors from rats which received 6 weekly intravesical instillations of 0.5 ml of PBS alone, or PBS containing 10⁸ CFU/ml of BCG vaccine or BCG Apa⁺⁺. Rats were euthanized at the 30^{th} week (one month after the last BCG treatment) and activated wild type p53 were measured by an Enzyme Immunometric Assay. **A.** Data reported well by well. **B.** Data reported as mean ± s.e.m.

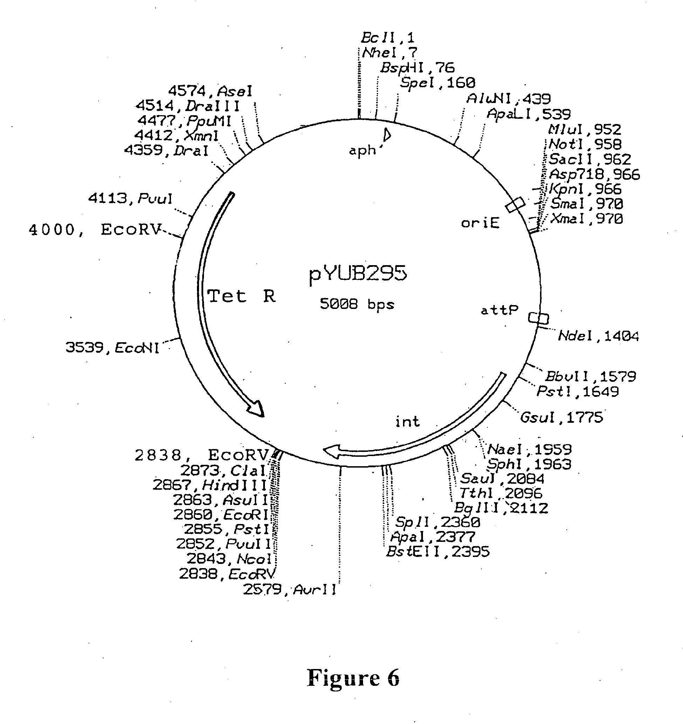

- Figure 6 is the map of the plasmid pYUB295

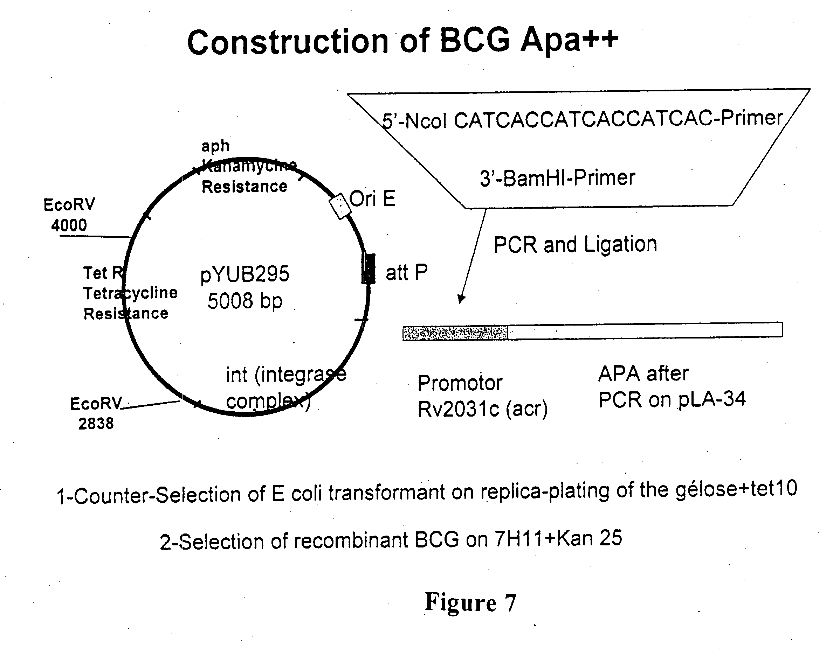

- Figure 7 illustrates the construction of the recombinant strain BCG Apa⁺⁺ containing the APA coding sequence under the transcriptional control of the HSPX promoter (derived from the pYAPA2031 plasmid), integrated into the BCG genome.

### Example 1: Construction of a recombinant BCG (BCG Apa⁺⁺) overexpressing the APA protein under the control of the alpha-crystallin promoter.

To construct a recombinant BCG able to produce APA molecules when the bacteria are under hypoxia, a plasmid containing the appropriate sequences was tailored. This plasmid contains the *apa* gene of *M. tuberculosis* (EMBL accession number X80268 and Laqueyrie et al., Infection and Immunity, 1995, 63, 4003-4010) coding for the APA protein, cloned under the transcriptional control of the HSPX (GenBank accession number S79751; Yuan et al., J. Bacteriol., 1996, 178, 4484-4492; Florczyk et al., Infect. Immun., 2001, 69, 5777-5785 and Florczyk et al., Infect. Immun., 2003, 71, 5332-5343), and the *aph* gene (kanamycin resistance). Bacteria of the wild BCG 1173P2 Pasteur strain, were then electroporated with the plasmid, and recombinant BCG overexpressing APA (BCG Apa⁺⁺) was isolated in Sauton medium containing kanamycin (Figure 7).

The *apa* DNA from *M. tuberculosis* was amplified by PCR from pLA34-2 (Laqueyrerie et al., Infect. Immun. 1995, 63, 4003-4010). The *apa* sequence was modified to create an *Nco*I site via the forward primer (5'-catgccatggtacaggtggaccccaacttgaca-3': SEQ ID NO: 1) and a *Bam*HI site in the reverse sequence (5'-ttaggatccggccggtaaggtccgctgcggtgt 3': SEQ ID NO: 2) in order to subclone the *Nco*I-*Bam*HI PCR product into the pQE60 expression vector (Qiagen; (Horn et al., J. Biol. Chem., 1999, 274, 32023-32030).

Two inducible BCG promoters with *Bam*HI and *Nco*I cohesive ends were generated by PCR performed on Pasteur *M. bovis* BCG DNA, using primers BamhspX F (5'-ttaggatccgtccggcatgatcaacctcc-3': SEQ ID NO: 3) and NcohspX R (5'-catgccatggggtggccatttgatgcctcc-3': SEQ ID NO: 4) for alpha-crystalline (*acr*) promoter and the primers BamRv2623 F (5'-ttaggatccgggccatggactggtcgtcg3': SEQ ID NO: 5) and NcoRV2623 R (5'-catgccatggacatcgcggtcctcctgtcg-3': SEQ ID NO: 6) for Rv2623 promoter (Florczyk et al., 2001 and 2003, precited).

These fragments were T4 ligated and after two Klenow treatments, *arc-*apa or Rv2623-apa cassettes were inserted into *EcoR*V restricted plasmid pYUB295 (Dussurget et al.,.Infect Immun., 2001, 69, 529-533; Gomez et al.,.Mol. Microbiol. 1998, 29, 617-628; Figure 6). This insertion created approximately 5.5 kbp plasmids of pYAPA2031 and pYAPA2623. After amplification in *E*. *coli* (XL-1 Blue) and tetracycline sensitivity replica plating, sensitive clones were selected. The plasmids were purified and electroporated into BCG. They do not replicate in BCG but contains the attachment site (attP) and the integrase gene (int) of the mycobacteriophage L5 that direct the integration of the plasmid at the attB site of the mycobacterial chromosome with high efficiency (Lee et al., Proc Natl Acad Sci USA, 1991, 29, 3111-3115). Low-oxygen liquid cultures were grown in vented-cap tissue culture flasks (25 cm² Coming) in an incubator which allowed to control the oxygen tension. Vented-cap tissue culture flasks allowed for the exchange of gases between the flasks and the controlled environment of the incubator. Oxygen levels were maintained by injecting prepared gas mixture (Carboxique) into the incubators. Low-oxygen cultures were grown under atmospheres of 1.3% total O₂. High-oxygen cultures were grown in ambient air in 25 cm² tissue culture flasks with the caps tightly sealed. When necessary, kanamycin was used at 25 µg/ml.

Overexpression was confirmed by Western blotting using a rabbit polyclonal antibody raised against the *M. bovis BCG Apa* (Laqueyrerie *et al*., Horn *et al*., precited).

### Example 2: Effect of the recombinant BCG Apa⁺⁺ on human carcinoma cell growth in vitro

### 1) Dimerization inhibition of FGFR3

The T24 cells derived from a human transitionnal cell carcinoma (ATCC number HTB-4), were incubated for 48 h without bacteria or with 10⁶ CFU/ml BCG vaccine or BCG Apa⁺⁺. The cells were cooled at 4°C during 15 min and treated for 2 h at 4°C in the absence or presence of 50 ng/ml of FGF-1 (PROMEGA). After covalent cross-linking with 1mM bis-(sulfosuccinimidyl)-suberate and 15 mM disuccinimidyl-suberate (PIERCE) for 30 min at room temperature, the cells were lysed, immunoprecipitated with anti-FGFR-3 C-terminal antibody (C-15), (SANTA CRUZ BIOTECHNOLOGY INC, sc-123), and probed on an immunoblot with a polyclonal antibody to the cytoplasmic domain of FGFR-3 (P-18) (SANTA CRUZ BIOTECHNOLOGY INC., sc-31162). The density obtained for the dimers (∼250 kDa) relative to the sum of the density of monomers (∼120 kDa) and dimers were determined.

The FGF receptor (FGFR-3) of the T24 carcinoma cells is activated by FGF. This activation is decreased in BCG vaccine treated cells and is totally abolished in BCG Apa⁺⁺ treated cells (figure 1). The inhibition of FGFR-3 dimerisation observed on cells stimulated with FGF-1 but incubated with BCG Apa⁺⁺, indicated that cells were inhibited in their growth.

### 2) Analysis of MAPK and Akt 1-2 pathways

Analysis of subsequent stimulation pathways was performed. Therefore, T24 cells were cultured in six-well plates. After 48 h incubation with 10⁶ CFU/ml BCG vaccine or BCG Apa⁺⁺ in serum-free DMEM, the cells were incubated for 15 minutes either without or with 100 ng/ml of EGF, and lysed. The presence of phosphorylated mitogen-activated protein kinase p42/p44 (pMAPK) and phosphorylated Akt 1-2 were determined by total protein ELISA by PhosphoDetect ERK 1/2 (pThr¹⁸⁵/pTyr¹⁸⁷) (CALBIOCHEM) and PhosphoDetect Akt (pSer⁴⁷³) (CALBIOCHEM) ELISA Kits respectively.

The results reporting phosphorylation of MAPK (figure 2) and Akt1/2 (figure 3) indicate that the presence of BCG Apa⁺⁺ on the T24 cell surface inhibits the first steps of FGF stimulation and confirm the tendency of BCG Apa⁺⁺ to be more potent than BCG to control *in vitro* the carcinoma cell growth.

### Example 3 : Effect of the recombinant BCG Apa⁺⁺ on human carcinoma cell growth in vivo

Thirty female Wistar rats (JANVIER), 7 weeks of age at the beginning of the experiment, were used in this study. N-butyl-N-(4-hydroxybutyl)nitrosamine BBN (KASEI INDUSTRY COMPANY) was given at 0.05 % in the drinking water of 24 rats for 19 weeks. 6 rats were maintained without any treatment up to 30 weeks. One week after BBN exposure treatment (20th week) the rats received 6 weekly intravesical instillations of 0.5 ml of PBS alone, or PBS containing 10⁸ CFU/ml of BCG vaccine or BCG Apa⁺⁺. Rats were euthanized at the 30th week (one month after the last BCG treatment). Bladders were removed, homogenized and nuclear protein extracts were prepared by Activemotif nuclear extract kit (Active motif, Carlsbad). Activated wild type p53 were measured by an Enzyme Immunometric Assay Kit (TiterZyme®, EIA900-117, Ann Arbor, MI).

BCG Apa⁺⁺ overexpressing APA molecules under mild hypoxia conditions observed in bladder was found to be more active than wild BCG to control carcinoma cell proliferation in bladder of rats receiving a chemical agent promoting carcinoma.

Clinical observation of the bladders indicated the presence of tumors in all the animals of the PBS-treated group and half of the animals of the BCG vaccine treated group. By contrast, the bladder was normal in all the animals of the BCG Apa⁺⁺-treated group (figure 4).

Analysis of wild-type p53 tumor suppressor factor concentration in the tumor cells nucleus, demonstrates no reduction in the BCG Apa⁺⁺-treated group, compared to a 50% reduction in the PBS-treated group and a 25% to 30% reduction in the BCG vaccine treated group (figure 5).

In these *in vivo* assays the results were more impressive than *in vitro,* perhaps due to a deeper hypoxia level in urine and bladder than in *in vitro* culture.

## Claims

1. A recombinant *Mycobacterium bovis* BCG strain, **characterized in that** it comprises a mycobacterial FAP protein coding sequence under the transcriptional control of a promoter sequence which is active under hypoxia conditions.

2. The recombinant BCG strain according to claim 1, **characterized in that** said mycobacterial FAP protein is the *Mycobacterium tuberculosis* APA protein.

3. The recombinant BCG strain according to claim 1 or claim 2, **characterized in that** said promoter is a mycobacterial promoter.

4. The recombinant BCG strain according to claim 3, **characterized in that** said promoter is the HSPX promoter.

5. The recombinant BCG strain according to anyone of claims 1 to 4, **characterized in that** it comprises a *Mycobacterium tuberculosis* APA protein coding sequence under the transcriptional control of the HSPX promoter.

6. The recombinant BCG strain according to anyone of claims 1 to 5, **characterized in that** it is stably transformed with the plasmid pYAPA2031, deposited at the Collection Nationale de Cultures de Microorganismes, 25 rue du Docteur Roux, 75724 Paris Cedex 15, on July 21 2006, under accession number I-3659, identified as BCG Apa⁺⁺.

7. An antitumoral composition, **characterized in that** it comprises a suitable amount of the recombinant BCG strain according to anyone of claims 1 to 6, in an acceptable carrier.

8. The antitumoral composition according to claim 7, **characterized in that** said recombinant BCG strain is living recombinant BCG.

9. The antitumoral composition according to claim 7, **characterized in that** said recombinant BCG strain is killed recombinant BCG.

10. The antitumoral composition according to claim 9, **characterized in that** said killed recombinant BCG is extended freeze-dried recombinant BCG.

11. The antitumoral composition according to anyone of claims 7 to 10, **characterized in that** it comprises between 10⁶ and 10¹⁰ CFU or CFU equivalent of recombinant BCG strain.

12. The antitumoral composition according to anyone of claims 7 to 11, **characterized in that** it comprises an immunostimulatory agent.

13. A product containing a recombinant BCG strain according to anyone of claims 1 to 6, and an immunostimulatory agent, as combined preparation for simultaneous, separate or sequential use in antitumoral therapy.

14. Use of a recombinant BCG strain according to anyone of claims 1 to 6, for the manufacture of a medicament intended for the prevention or the treatment of a tumor in a subject.

15. The use according to claim 14, **characterized in that** said medicament is intended for the treatment of epithelial tumors.

16. The use according to claim 14 or claim 15, **characterized in that** said tumors are selected from the group consisting of: transitional cell carcinoma of the bladder, lung carcinoma, cervix carcinoma and colon carcinoma.

17. The use according to anyone of claims 14 to 16, **characterized in that** recombinant BCG strain is the BCG Apa⁺⁺ strain.

18. Use of an expression cassette consisting of a polynucleotide fragment comprising a promoter sequence which is active under hypoxia conditions, as defined in any one of claims 1, 3 and 4, operatively linked to a mycobacterial FAP protein coding sequence, as defined in claim 1 or claim 2 or of a recombinant vector comprising said polynucleotide fragment, for the preparation of a recombinant *Mycobacterium bovis* BCG strain according to anyone of claims I to 6.

19. The use according to claim 18, **characterized in that** said recombinant vector is a recombinant plasmid.

20. The use according to claim 18, **characterized in that** the expression cassette is integrated in the genome of said recombinant *Mycobacterium bovis* BCG strain.

## Patentansprüche

1. Rekombinanter *Mycobacterium bovis* BCG-Stamm, **dadurch gekennzeichnet, dass** er eine ein mycobakterielles FAP-Protein codierende Sequenz unter der transkriptionellen Kontrolle einer Promotorsequenz, welche unter hypoxischen Bedingungen aktiv ist, umfasst.

2. Rekombinanter BCG-Stamm gemäß Anspruch 1, **dadurch gekennzeichnet, dass** das mycobakterielle FAP-Protein das *Mycobacterium tuberculosis* APA-Protein ist.

3. Rekombinanter BCG-Stamm gemäß Anspruch 1 oder Anspruch 2, **dadurch gekennzeichnet, dass** der Promotor ein mycobakterieller Promoter ist.

4. Rekombinanter BCG-Stamm gemäß Anspruch 3, **dadurch gekennzeichnet, dass** der Promotor der HSPX-Promotor ist.

5. Rekombinanter BCG-Stamm gemäß einem der Ansprüche 1 bis 4, **dadurch gekennzeichnet, dass** er eine *Mycobacterium tuberculosis* APA-Protein-codierende Sequenz unter der transkriptionellen Kontrolle des HSPX-Promotors umfasst.

6. Rekombinanter BCG-Stamm gemäß einem der Ansprüche 1 bis 6, **dadurch gekennzeichnet, dass** er mit dem Plasmid pYAPA2031, hinterlegt bei der Collection Nationale de Cultures de Microorganismes, 25 rue du Docteur Roux, 75724 Paris Cedex 15 am 21. Juli 2006 mit der Hinterlegungsnummer 1-3659, stabil transformiert ist, bezeichnet als BCG Apa⁺⁺.

7. Antitumorale Zusammensetzung, **dadurch gekennzeichnet, dass** sie eine geeignete Menge des rekombinanten BCG-Stamms gemäß einem der Ansprüche 1 bis 6 in einem annehmbaren Träger umfasst.

8. Antitumorale Zusammensetzung gemäß Anspruch 7, **dadurch gekennzeichnet, dass** der rekombinante BCG-Stamm ein lebender rekombinanter BCG-Stamm ist.

9. Antitumorale Zusammensetzung gemäß Anspruch 7, **dadurch gekennzeichnet, dass** der rekombinante BCG-Stamm ein abgetöteter BCG-Stamm ist.

10. Antitumorale Zusammensetzung gemäß Anspruch 9, **dadurch gekennzeichnet, dass** der abgetötete rekombinante BCG-Stamm ein ausgedehnt gefriergetrockneter rekombinanter BCG-Stamm ist.

11. Antitumorale Zusammensetzung gemäß einem der Ansprüche 7 bis 10, **dadurch gekennzeichnet, dass** sie zwischen 10⁶ und 10¹⁰ KbE oder KbE-Äquivalent des rekombinanten BCG-Stamms umfasst.

12. Antitumorale Zusammensetzung gemäß einem der Ansprüche 7 bis 11, **dadurch gekennzeichnet, dass** sie ein immunstimulatorisches Agens umfasst.

13. Produkt, enthaltend einen rekombinanten BCG-Stamm gemäß einem der Ansprüche 1 bis 6 und ein immunstimulatorisches Agens als kombinierte Zubereitung zur simultanen, separaten oder sequentiellen Verwendung in einer antitumoralen Therapie.

14. Verwendung eines rekombinanten BCG-Stamms gemäß einem der Ansprüche 1 bis 6 zur Herstellung eines Medikaments zum Zweck der Prävention oder der Behandlung eines Tumors in einem Subjekt.

15. Verwendung gemäß Anspruch 14, **dadurch gekennzeichnet, dass** das Medikament zur Behandlung von epithelialen Tumoren gedacht ist.

16. Verwendung gemäß Anspruch 14 oder Anspruch 15, **dadurch gekennzeichnet, dass** die Tumore ausgewählt sind aus der Gruppe, bestehend aus Übergangsepitelkarzinom der Blase, Lungenkarzinom, Cervixkarzinom und Kolonkarzinom.

17. Verwendung gemäß einem der Ansprüche 14 bis 16, **dadurch gekennzeichnet, dass** der rekombinante BCG-Stamm der BCG Apa⁺⁺-Stamm ist.

18. Verwendung einer Expressionskassette, bestehend aus einem Polynucleotidfragment, umfassend eine Promotorsequenz, welche unter hypoxischen Bedingungen aktiv ist, wie in einem der Ansprüche 1, 3 und 4 definiert, funktionell verknüpft mit einer mycobakterielles FAP-Protein codierenden Sequenz, wie in Anspruch 1 oder Anspruch 2 definiert, oder eines rekombinanten Vektors, umfassend das Polynucleotidfragment zur Herstellung eines rekombinanten *Mycobacterium bovis* BCG-Stamms gemäß einem der Ansprüche 1 bis 6.

19. Verwendung gemäß Anspruch 18, **dadurch gekennzeichnet, dass** der rekombinante Vektor ein rekombinantes Plasmid ist.

20. Verwendung gemäß Anspruch 18, **dadurch gekennzeichnet, dass** die Expressionskassette in das Genom des rekombinanten *Mycobacterium bovis* BCG-Stamms integriert ist.

## Revendications

1. Souche de *Mycobacterium bovis* BCG recombinante, **caractérisée en ce qu'**elle comprend une séquence codant pour une protéine FAP mycobactérienne sous le contrôle transcriptionnel d'une séquence de promoteur qui est active dans des conditions d'hypoxie.

2. Souche de BCG recombinante selon la revendication 1, **caractérisée en ce que** ladite protéine FAP mycobactérienne est la protéine APA de *Mycobacterium tuberculosis.*

3. Souche de BCG recombinante selon la revendication 1 ou la revendication 2, **caractérisée en ce que** ledit promoteur est un promoteur mycobactérien.

4. Souche de BCG recombinante selon la revendication 3, **caractérisée en ce que** ledit promoteur est le promoteur HSPX.

5. Souche de BCG recombinante selon l'une quelconque des revendications 1 à 4, **caractérisée en ce qu'**elle comprend une séquence codant pour la protéine APA de *Mycobacterium tuberculosis* sous le contrôle transcriptionnel du promoteur HSPX.

6. Souche de BCG recombinante selon l'une quelconque des revendications 1 à 5, **caractérisée en ce qu'**elle est transformée de façon stable avec le plasmide pYAPA2031, déposé auprès de la Collection Nationale de Cultures de Microorganismes, 25 rue du Docteur Roux, 75724 Paris Cedex 15, le 21 juillet 2006, sous le numéro d'accession 1-3659, identifié sous le nom BCG Apa⁺⁺.

7. Composition antitumorale, **caractérisée en ce qu'**elle comprend une quantité adaptée de la souche de BCG recombinante selon l'une quelconque des revendications 1 à 6, dans un véhicule acceptable.

8. Composition antitumorale selon la revendication 7, **caractérisée en ce que** ladite souche de BCG recombinante est un BCG recombinant vivant.

9. Composition antitumorale selon la revendication 7, **caractérisée en ce que** ladite souche de BCG recombinante est un BCG recombinant tué.

10. Composition antitumorale selon la revendication 9, **caractérisée en ce que** ledit BCG recombinant tué est un BCG recombinant lyophilisé de manière extensive.

11. Composition antitumorale selon l'une quelconque des revendications 7 à 10, **caractérisée en ce qu'**elle comprend entre 10⁶ et 10¹⁰ UFC ou équivalents d'UFC de souche de BCG recombinante.

12. Composition antitumorale selon l'une quelconque des revendications 7 à 11, **caractérisée en ce qu'**elle comprend un agent immunostimulant.

13. Produit contenant une souche de BCG recombinante selon l'une quelconque des revendications 1 à 6, et un agent immunostimulant, sous forme de préparation combinée pour utilisation simultanée, séparée ou séquentielle en thérapie antitumorale.

14. Utilisation d'une souche de BCG recombinante selon l'une quelconque des revendications 1 à 6, pour la fabrication d'un médicament destiné à la prévention ou au traitement d'une tumeur chez un sujet.

15. Utilisation selon la revendication 14, **caractérisée en ce que** ledit médicament est destiné au traitement de tumeurs épithéliales.

16. Utilisation selon la revendication 14 ou la revendication 15, **caractérisée en ce que** lesdites tumeurs sont choisies dans le groupe constitué de : un carcinome à cellules transitionnelles de la vessie, un carcinome du poumon, un carcinome du col de l'utérus et un carcinome du côlon.

17. Utilisation selon l'une quelconque des revendications 14 à 16, **caractérisée en ce que** la souche de BCG recombinante est la souche BCG Apa⁺⁺.

18. Utilisation d'une cassette d'expression constituée d'un fragment de polynucléotide comprenant une séquence de promoteur qui est active dans des conditions d'hypoxie, tel que défini dans l'une quelconque des revendications 1, 3 et 4, fonctionnellement liée à une séquence codant pour une protéine FAP mycobactérienne, telle que définie dans la revendication 1 ou la revendication 2 ou d'un vecteur recombinant comprenant ledit fragment de polynucléotide, pour la préparation d'une souche de *Mycobacterium bovis* BCG recombinante selon l'une quelconque des revendications 1 à 6.

19. Utilisation selon la revendication 18, **caractérisée en ce que** ledit vecteur recombinant est un plasmide recombinant.

20. Utilisation selon la revendication 18, **caractérisée en ce que** la cassette d'expression est intégrée dans le génome de ladite souche de *Mycobacterium bovis* BCG recombinante.
